# EUROPEAN PATENT APPLICATION

(11) **EP 3 949 918 A1**
(43) Date of publication of application: **09.02.2022**
(21) Application number: 20868082.7
(22) Date of filing: 08.07.2020
(51) Int. Cl.: A61F 7/00, A61L 15/20

(54) **HOT AND COLD PACK PRODUCT, AND PREPARATION METHOD FOR SAME**

(30) Priority: 29.09.2019 CN 201910936546
(71) Applicant: Jiangsu Yishun Medical Equipment Co., Ltd, Nantong, Jiangsu 226200 (CN)
(72) Inventor: ZHANG, Zhi, Qidong City, Nantong, Jiangsu 226200 (CN)
(74) Representative: Petculescu, Ana-Maria
(86) International application number: PCT/CN2020/100780
(87) International publication number: WO 2021/057177

(57) **Abstract**

The examples of the present application disclose a cold and hot compress product and a preparation method therefor, which relate to the technical field of life and health care products; the preparation method includes the following steps: weighing the following raw materials by weight percentages: 5%-25% of acrylamide, 1%-10% of methylol acrylamide, 15-35% of glycerol, 0.1%-1% of sodium chloride, and 30%-50% of water; firstly adding water and glycerol to a reaction kettle, then adding acrylamide, methylol acrylamide and sodium chloride, and then stirring same together for 12-36 hours to obtain a stock material; filling the stock material in an outer bag, and pressing the outer bag containing the stock material with a flat weight for 30 to 180 minutes to form an elastic gel body in the outer bag; removing the outer bag and wrapping the elastic gel body with a wrapping cloth, and edge-binding the wrapping cloth along the shape of the elastic gel body. The preparation method is simple, with the raw materials being cheap and easily available, and having no pollution, and is easy to popularize; the prepared cold and hot compress products have excellent heat preservation and cold storage properties, and are not easily deformed.

## Description

The present application claims the priority to Chinese Patent Application No. 201910936546.0, titled "Cold and Hot Compress Product and Preparation Method Therefor", filed with the China National Intellectual Property Administration on September 29, 2019, which is incorporated herein by reference in its entirety.

### Technical Field

The examples of the present application relate to the technical field of life and health care products, in particular to a cold and hot compress product and a preparation method therefor.

### Background Art

Cold and hot compress is the most commonly used treatment method to relieve discomfort in various parts of the human body; for example, the discomfort in shoulders, neck, chest, back, waist, abdomen, wrists, elbows, knees, and feet, etc., of the human body can be relieved by cold compress or hot compress, which is also a very common treatment method in the medical process; whether it is muscle injuries caused by exercise, traumatic injuries, inflammation and detumescence, or promoting blood circulation to remove blood stasis, both cold compress and hot compress can alleviate the discomfort.

Media for traditional cold and hot compress mostly are towels or hot-water bags, one is cold and the other one is hot, the two alternate with each other when used, which is not convenient; and since the iced towel and hot water bag are completely exposed to the air, and the medium material is not specially structured, the energy storage time of the traditional cold and hot compress method is short, lacking the effect durability. At present, cold and hot compress products formed by using hydrogels as dressings to be put in outer bags are also sold on the market, but the hydrogel dressings are easy to flow during use, resulting in a poor cold and hot compress effect, and dressings tend to accumulate to the bottom, which cannot achieve the effect of uniform cold and hot compress.

### Summary of the Invention

For this reason, the examples of the present application provide a cold and hot compress product and a preparation method therefor, to solve the problems of the existing cold and hot compress product that has the problems of a poor cold and hot compress effect and the ease of accumulation of the dressings, etc.

In order to achieve the above object, the examples of the present application provide the following technical solutions:
According to a first aspect of the examples of the present application, a preparation method for a cold and hot compress product, includes the following steps:
step 1: weighing the following raw materials by weight percentages: 5%-25% of acrylamide, 1%-10% of methylol acrylamide, 15%-35% of glycerol, 0.1%-1% of sodium chloride, and 30%-50% of water;
step 2: firstly adding water and glycerol to a reaction kettle, then adding acrylamide, methylol acrylamide and sodium chloride, and then stirring same together for 12-36 hours to obtain a stock material;
step 3: filling the stock material obtained in step 2 in an outer bag, and pressing the outer bag containing the stock material with a flat weight for 30 to 180 minutes to form an elastic gel body in the outer bag;
step 4: removing the outer bag and wrapping the elastic gel body with a wrapping cloth, and edge-binding the wrapping cloth along the shape of the elastic gel body to obtain the cold and hot compress product.

Further, the preparation method further includes:
step 5: carrying out quality inspection on the cold and hot compress product obtained in step 4, and packing the accepted products into the warehouses.

Further, the shapes of the outer bag and the wrapping cloth match the overall dimension of the cold and hot compress product.

Further, the wrapping cloth is an elastic cloth.

Further, the elastic cloth is a lycra cloth.

Further, the outer bag is formed by stacking two pieces of PVC materials together and heat sealing same.

Further, the flat weight is an aluminium plate.

Further, in step 1, the weight percentages of the various raw materials are: 18% of acrylamide, 3.6% of methylol acrylamide, 30% of glycerol, 0.4% of sodium chloride, and 48% of water.

According to a second aspect of the examples of the present application, a cold and hot compress product, is prepared by the preparation method according to the above claims.

The cold and hot compress product is a cold and hot compress elbow pad, a cold and hot compress shoulder pad, a cold and hot compress eye shield, or a cold and hot compress pad.

The examples of the present application have the following advantages:
the preparation method of a cold and hot compress product according to the examples of the present application is simple, with the raw materials being cheap and easily available, and having no pollution, and is easy to popularize; the cold and hot compress product prepared by said preparation method has excellent heat preservation and cold storage properties, good cooling sensation, and the prepared elastic gel body of the dressing has good elasticity and is not easily deformed, so that the cold and hot compress product can maintain the original shape all the time without shrinking.

### Brief Description of the Drawings

To describe the technical solutions in the implementations of the present application or in the prior art more clearly, the following briefly introduces the accompanying drawings required for describing the implementations or the prior art. It is obvious that the drawings in the following description are merely exemplary, and for those of ordinary skill in the art, other implementation drawings would have been derived from the extension of the provided drawings without involving any inventive effort.

The structures, proportions, sizes and the like depicted in this specification are only used to fit with the content disclosed in the specification for understanding and reading by those skilled in the art, but are not intended to limit the limitation conditions that can be implemented in the present application, and thus have no technically substantive meaning. Any of the modification of structure, the change of proportional relationship or the adjustment of size should fall within the scope covered by the technical content disclosed in the present application without affecting the effects produced and the object achievable by the present application.

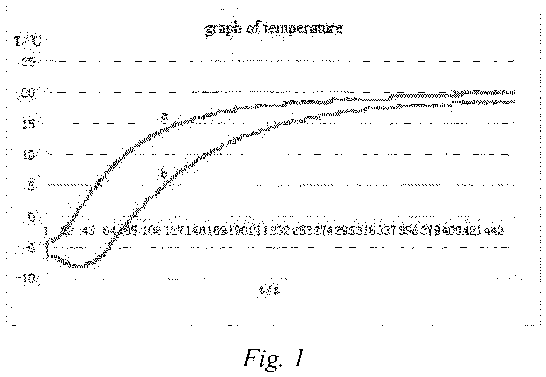

FIG. 1 is a comparison diagram of temperature change curves of a sample of comparative example and a sample of test example provided by an experimental example of the present application.

### Detailed Description of Embodiments

The examples of the present application are illustrated below by using the specific embodiments, and those skilled in the art would have readily understood other advantages and effects of the present application from the disclosure of this description, and it is obvious that the described examples are some of the examples of the present application rather than all the examples. Based on the examples of the present application, all other examples obtained by those of ordinary skill in the art without involving any inventive effort fall within the scope of protection of the present application.

### Example 1

A preparation method of a cold and hot compress product includes the following steps:
step 1: weighing the following raw materials: 90 kg of acrylamide, 18 kg of methylol acrylamide, 150 kg of glycerol, 2 kg of sodium chloride, and 240 kg of purified water;
step 2: firstly adding purified water and glycerol to the reaction kettle, then adding acrylamide, methylol acrylamide and sodium chloride, and afterwards stirring same together for 24 hours to obtain a stock material;
step 3: filling the stock material obtained in step 2 in an outer bag, and pressing the outer bag containing the stock material with an aluminium plate for 120 minutes to form an elastic gel body in the outer bag;
step 4: removing the outer bag and wrapping the elastic gel body with a wrapping cloth, and edge-binding the wrapping cloth along the shape of the elastic gel body to obtain the cold and hot compress product.
step 5: carrying out quality inspection on the cold and hot compress product obtained in step 4, and packing the accepted products into the warehouses.

The shapes of the outer bag and the wrapping cloth match the overall dimension of the cold and hot compress product, the wrapping cloth is a lycra cloth, and the outer bag is formed by stacking two pieces of PVC materials together and heat sealing with a high frequency machine. In such a case, the PVC material and lycra cloth are cut into a trapezoid shape with an upper 250 mm, a lower 350 mm and a height of 260 mm;

The cold and hot compress product is a cold and hot compress elbow pad, and a cold and hot compress shoulder pad.

### Example 2

A preparation method of a cold and hot compress product includes the following steps:
step 1: weighing the following raw materials: 50 g of acrylamide, 100 g of methylol acrylamide, 340 g of glycerol, 10 g of sodium chloride, and 500 g of purified water;
step 2: firstly adding purified water and glycerol to the reaction kettle, then adding acrylamide, methylol acrylamide and sodium chloride, and afterwards stirring same together for 12 hours to obtain a stock material;
step 3: filling the stock material obtained in step 2 in an outer bag, and pressing the outer bag containing the stock material with an aluminium plate for 30 minutes to form an elastic gel body in the outer bag;
step 4: removing the outer bag and wrapping the elastic gel body with a wrapping cloth, and edge-binding the wrapping cloth along the shape of the elastic gel body to obtain the cold and hot compress product.
step 5: carrying out quality inspection on the cold and hot compress product obtained in step 4, and packing the accepted products into the warehouses.

The shapes of the outer bag and the wrapping cloth match the overall dimension of the cold and hot compress product, the wrapping cloth is a lycra cloth, and the outer bag is formed by stacking two pieces of PVC materials together and heat sealing with a high frequency machine.

The cold and hot compress product is a cold and hot compress eye shield.

### Example 3

A preparation method of a cold and hot compress product includes the following steps:
step 1: weighing the following raw materials: 210 g of acrylamide, 12 g of methylol acrylamide, 270 g of glycerol, 8 g of sodium chloride, and 500 g of purified water;
step 2: firstly adding purified water and glycerol to the reaction kettle, then adding acrylamide, methylol acrylamide and sodium chloride, and afterwards stirring same together for 12 hours to obtain a stock material;
step 3: filling the stock material obtained in step 2 in an outer bag, and pressing the outer bag containing the stock material with an aluminium plate for 60 minutes to form an elastic gel body in the outer bag;
step 4: removing the outer bag and wrapping the elastic gel body with a wrapping cloth, and edge-binding the wrapping cloth along the shape of the elastic gel body to obtain the cold and hot compress product.
step 5: carrying out quality inspection on the cold and hot compress product obtained in step 4, and packing the accepted products into the warehouses.

The shapes of the outer bag and the wrapping cloth match the overall dimension of the cold and hot compress product, the wrapping cloth is a lycra cloth, and the outer bag is formed by stacking two pieces of PVC materials together and heat sealing with a high frequency machine.

The cold and hot compress product is a cold and hot compress eye shield.

### Example 4

A preparation method of a cold and hot compress product includes the following steps:
step 1: weighing the following raw materials: 250 kg of acrylamide, 99 kg of methylol acrylamide, 150 kg of glycerol, 1 kg of sodium chloride, and 500 kg of purified water;
step 2: firstly adding purified water and glycerol to the reaction kettle, then adding acrylamide, methylol acrylamide and sodium chloride, and afterwards stirring same together for 36 hours to obtain a stock material;
step 3: filling the stock material obtained in step 2 in an outer bag, and pressing the outer bag containing the stock material with an aluminium plate for 90 minutes to form an elastic gel body in the outer bag;
step 4: removing the outer bag and wrapping the elastic gel body with a wrapping cloth, and edge-binding the wrapping cloth along the shape of the elastic gel body to obtain the cold and hot compress product.
step 5: carrying out quality inspection on the cold and hot compress product obtained in step 4, and packing the accepted products into the warehouses.

The shapes of the outer bag and the wrapping cloth match the overall dimension of the cold and hot compress product, the wrapping cloth is a lycra cloth, and the outer bag is formed by stacking two pieces of PVC materials together and heat sealing with a high frequency machine.

The cold and hot compress product is a cold and hot compress pad.

### Example 5

A preparation method of a cold and hot compress product includes the following steps:
step 1: weighing the following raw materials: 250 kg of acrylamide, 90 kg of methylol acrylamide, 350 kg of glycerol, 10 kg of sodium chloride, and 300 kg of purified water;
step 2: firstly adding purified water and glycerol to the reaction kettle, then adding acrylamide, methylol acrylamide and sodium chloride, and afterwards stirring same together for 36 hours to obtain a stock material;
step 3: filling the stock material obtained in step 2 in an outer bag, and pressing the outer bag containing the stock material with an aluminium plate for 180 minutes to form an elastic gel body in the outer bag;
step 4: removing the outer bag and wrapping the elastic gel body with a wrapping cloth, and edge-binding the wrapping cloth along the shape of the elastic gel body to obtain the cold and hot compress product.
step 5: carrying out quality inspection on the cold and hot compress product obtained in step 4, and packing the accepted products into the warehouses.

The shapes of the outer bag and the wrapping cloth match the overall dimension of the cold and hot compress product, the wrapping cloth is a lycra cloth, and the outer bag is formed by stacking two pieces of PVC materials together and heat sealing with a high frequency machine.

The cold and hot compress product is a cold and hot compress pad.

### Experimental Example

1. Experimental method:
   The cold and hot compress bag on the market that comprises a hydrogel as a dressing is taken as comparative examples, and the cold and hot compress product prepared in Example 1 is taken as a test example for test analysis, the sample of the comparative example and the sample of the test example are put into a refrigerator at -18 degree to be frozen for 8 hours, respectively, then taken out, the well-set temperature recorders are fixed at the bottom in the middle of the samples under the conditions of ambient temperature of 20°C, respectively; the temperature change of the sample surface with time is tested, and the comparison diagram of the temperature change curve is shown in the FIG.1, wherein curve a shows the temperature change curve of the sample of the comparative examples, and curve b shows the temperature change curve of the sample of the test examples.
2. Analysis of Experiment
   It can be seen from FIG. 1 that the surface temperature of the sample is -6.5°C when the sample of the comparative example and the sample of the test example are taken out of the refrigerator, and the surface temperature of the sample of the comparative examples gradually rises to 20°C with time, whereas the surface temperature of the sample of the test example first is maintained at -6.5°C for a period of time, then gradually decreased to -8°C, and then maintained at -8°C for a period of time again, and then gradually increased to 18.5°C again. It shows that the sample of the test example of the present application has excellent heat retention and cold storage properties, and then the sample of this example is subjected to the above-mentioned test for 20 cycles, and the sample of the test example of the present application still have high elasticity and do not deform.

Although the present application has been described in detail above with the general description and particular examples, on the basis of the present application, some modifications or improvements can be made thereto, which would have been obvious to those skilled in the art. Therefore, all the modifications and improvements which can be made without departing from the spirit of the present application belong to the scope of protection of the present application.

## Claims

1. A preparation method for a cold and hot compress product, **characterized in that**, the preparation method includes the following steps:
step 1: weighing the following raw materials by weight percentages: 5%-25% of acrylamide, 1%-10% of methylol acrylamide, 15%-35% of glycerol, 0.1%-1% of sodium chloride, and 30%-50% of water;
step 2: firstly adding water and glycerol to a reaction kettle, then adding acrylamide, methylol acrylamide and sodium chloride, and then stirring same together for 12-36 hours to obtain a stock material;
step 3: filling the stock material obtained in step 2 in an outer bag, and pressing the outer bag containing the stock material with a flat weight for 30 to 180 minutes to form an elastic gel body in the outer bag;
step 4: removing the outer bag and wrapping the elastic gel body with a wrapping cloth, and edge-binding the wrapping cloth along the shape of the elastic gel body to obtain the cold and hot compress product.

2. A preparation method for a cold and hot compress product according to claim 1, **characterized in that** the preparation method further includes:
step 5: carrying out quality inspection on the cold and hot compress product obtained in step 4, and packing the accepted products into the warehouses.

3. A preparation method for a cold and hot compress product according to claim 1, **characterized in that** the shapes of the outer bag and the wrapping cloth match the overall dimension of the cold and hot compress product.

4. A preparation method for a cold and hot compress product according to claim 3, **characterized in that** the wrapping cloth is an elastic cloth.

5. A preparation method for a cold and hot compress product according to claim 4, **characterized in that** the elastic cloth is a lycra cloth.

6. A preparation method for a cold and hot compress product according to claim 3, **characterized in that** the outer bag is formed by stacking two pieces of PVC materials together and heat sealing same.

7. A preparation method for a cold and hot compress product according to claim 1, **characterized in that** the flat weight is an aluminium plate.

8. A preparation method for a cold and hot compress product according to claim 1, **characterized in that** in step 1, the weight percentages of the various raw materials are: 18% of acrylamide, 3.6% of methylol acrylamide, 30% of glycerol, 0.4% of sodium chloride, and 48% of water.

9. A cold and hot compress product, **characterized in that** the cold and hot compress product is prepared by the preparation method according to any one of claims 1-8.

10. A cold and hot compress product according to claim 9, **characterized in that** the cold and hot compress product is a cold and hot compress elbow pad, a cold and hot compress shoulder pad, a cold and hot compress eye shield, or a cold and hot compress pad.
